(19) Europäisches Patentamt
European Patent Office
Office européen des brevets

(11) EP 0 939 074 A2

(12) **EUROPÄISCHE PATENTANMELDUNG**

(43) Veröffentlichungstag:
**01.09.1999 Patentblatt 1999/35**

(51) Int. Cl.[6]: **C07C 209/60**, C07F 9/50, C07F 15/00

(21) Anmeldenummer: **99102931.5**

(22) Anmeldetag: **13.02.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **27.02.1998 DE 19808260**

(71) Anmelder:
**Bayer Aktiengesellschaft**
**51368 Leverkusen (DE)**

(72) Erfinder:
- **Traenckner, Hans-Joachim, Dr.**
  **51467 Bergisch Gladbach (DE)**
- **Jentsch, Jörg-Dietrich, Dr.**
  **47799 Krefeld (DE)**
- **Prinz, Thomas, Dr.**
  **50753 Köln (DE)**
- **Driessen-Hölscher, Birgit, Dr.**
  **52074 Aachen (DE)**
- **Keim, Wilhelm, Prof. Dr.**
  **52074 Aachen (DE)**

(54) **Verbessertes Verfahren zur Herstellung von Octa-2,7-dienyl-1-amin aus Butadien und Ammoniak in Gegenwart von Palladium-Komplexkatalysatoren**

(57) Verfahren zur Herstellung von Octa-2,7-dienyl-1-amin durch Telomerisation von Butadien mit Ammoniak in einem Zweiphasensystem in Gegenwart von hydrophilen Palladiumkomplexen, dadurch gekennzeichnet, daß man aus dem nach der Telomerisation vorliegenden Reaktionsgemisches getrennt voneinander Octa-2,7-dienyl-1-amin und Octa-1,7-dienyl-3-amin isoliert und das so isolierte Octa-1,7-dienyl-3-amin einer Isomerisierung unter Bildung von Octa-2,7-dienyl-1-amin unterwirft, sowie neue Triphenylphosphanmono- und -dimethoxytrinatriumsulfonate und die Verwendung von Triphenyltrimethoxytrinatriumsulfonaten, Triphenylphosphantrimethyltrinatriumsulfonaten und Triphenylphosphantrifluordinatrium- und -trinatriumsulfonaten als Liganden zur Herstellung von Palladium-Komplexen.

EP 0 939 074 A2

## Beschreibung

Die vorliegende Erfindung betrifft ein verbessertes Verfahren zur Herstellung von Octa-2,7-dienyl-1-amin durch Telomerisation von Butadien mit Ammoniak in einem Zweiphasensystem, anschließender Isolierung von Octa-2,7-dienyl-1-amin und Octa-1,7-dienyl-3-amin aus dem Reaktionsgemisch und Isomerisierung des Octa-1,7-dienyl-3-amins zu Octa-2,7-dienyl-1-amin. Die vorliegende Erfindung betrifft weiterhin neue Triphenylphosphanmono- und -dimethoxytrinatriumsulfonate und die Verwendung von Triphenyltrimethoxytrinatriumsulfonaten, Triphenylphosphantrimethyltrinatriumsulfonaten und Triphenylphosphantrifluordinatrium- und -trinatriumsulfonaten als Liganden zur Herstellung von Palladium-Komplexen.

Octadienylamine sind z.B. als Zwischenprodukte zur Herstellung von Octylaminen verwendbar, die ihrerseits z.B. zur Herstellung von Weichspülern, Korrosionsinhibitoren, Flotationshilfsmitteln und Emulgatoren benötigt werden. Für diese Zwecke ist insbesondere Octa-2,7-dienyl-1-amin von Interesse.

Üblicherweise fallen bei der Telomerisierung von Butadien und Ammoniak komplexe Reaktionsgemische an, die neben Octa-2,7-dienyl-1-amin erhebliche Mengen Octa-1,7-dienyl-3-amin und sekundäre und tertiäre Octadienylamine enthalten.

Aus der EP-A 773 211 ist bekannt, die Telomerisation von Butadien und Ammoniak in einem Zweiphasensystem durchzuführen und als Katalysatoren Gemische von Palladiumverbindungen und diese besser wasserlöslich machenden Phosphorverbindungen, etwa sulfonierte Triphenylphosphane, einzusetzen. Man erhält Reaktionsgemische, die Octa-2,7-dienyl-1-amin (1), Octa-1,7-dienyl-3-amin (2) und sekundäre Octadienylamine (3) enthalten. Dabei bildet sich (1) in Selektivitäten von 40 bis 56 %, (2) in Selektivitäten von 28 bis 43 % und (3) in Selektivitäten von bis zu 10 %. Die Reaktionsgemische wurden nicht aufgearbeitet.

Gemäß der EP-A 816 308 werden bei der besagten Telomerisation als Katalysatoren Komplexverbindungen eingesetzt, die als Zentralatom ein Übergangsmetall und wenigstens ein Tris(hydroxyalkyl)-phosphan oder -phosphanoxid als Ligand enthalten. Bei Butadienumsätzen von 4 bis 63 % läßt sich die Selektivität der Bildung von (1) bis auf 66 % steigern, bei Butadienumsätzen unter 2 % (d.h. großen Butadienüberschüssen) sogar bis auf 85 %. Die Selektivität der Bildung von (2) liegt im Bereich 1 bis 40 %, wobei keine direkte Relation der Selektivitäten der Bildung von (1) und (2) besteht. Beispielsweise wird (2) mit einer Selektivität von 9 % erhalten, wenn (1) mit einer Selektivität von 85 % anfällt und in einer Selektivität von 1 %, wenn (1) mit einer Selektivität von 55 % anfällt. Die Reaktionsgemische sind auch hier nicht aufgearbeitet worden.

Es besteht deshalb noch ein Bedürfnis nach einem Verfahren, mit dem Octa-2,7-dienyl-1-amin möglichst unabhängig von den verwendeten Katalysatoren, Butadienüberschüssen und sonstigen Parametern in guten Selektivitäten und Ausbeuten zugänglich ist.

Es wurde nun ein Verfahren zur Herstellung von Octa-2,7-dienyl-1-amin durch Telomerisation von Butadien mit Ammoniak in einem Zweiphasensystem in Gegenwart von hydrophilen Palladiumkatalysatoren gefunden, das dadurch gekennzeichnet ist, daß man aus dem nach der Telomerisation vorliegenden Reaktionsgemisch getrennt voneinander Octa-2,7-dienyl-1-amin und Octa-1,7-dienyl-3-amin isoliert und das so isolierte Octa-1,7-dienyl-3-amin einer Isomerisierung unter Bildung von Octa-2,7-dienyl-1-amin unterwirft.

Ammoniak kann man in beliebiger Form einsetzen. Vorzugsweise verwendet man Mischungen von Ammoniak und Wasser, beispielsweise 5 bis 35 gew.-%ige wäßrige Ammoniaklösungen, oder reinen Ammoniak (Handelsprodukt).

Das Zweiphasensystem besteht im allgemeinen aus einer hydrophilen und einer hydrophoben Phase. Die hydrophile Phase kann dabei als wesentliche Komponente, z.B. Wasser oder ein hydrophiles Lösungsmittel oder ein Gemisch aus hydrophilem Lösungsmittel und Wasser enthalten. Bei den hydrophilen Lösungsmitteln kann es sich z.B. um $C_1$-$C_6$-Mono-, Di-, Tri-, Tetra-, Penta- und Hexaalkohole, um Tetramethylensulfon, um Dimethylsulfoxid oder um Acetonitril handeln. Als hydrophile Phase wird vorzugsweise Wasser verwendet, das z.B. als solches und/oder in Form wäßriger Lösungen von Ammoniak eingesetzt werden kann.

Die hydrophobe Phase kann als wesentliche Komponente überschüssiges Butadien und/oder ein mit Wasser nicht oder nur wenig mischbares organisches Lösungsmittel enthalten. Als mit Wasser nicht oder nur wenig mischbare organische Lösungsmittel kommen z.B. solche in Frage, von denen sich bei 20°C in 100 g Wasser nur weniger als 5 g, insbesondere weniger als 3 g lösen. Beispiele für solche Lösungsmittel sind unter Reaktionsbedingungen flüssige aliphatische und aromatische Kohlenwasserstoffe, chlorierte aliphatische und aromatische Kohlenwasserstoffe, Ether, tert.-Amine und Pyrrolidon. Als hydrophobe Phase wird vorzugsweise Butadien, Benzol, Toluol oder Methylenchlorid verwendet, insbesondere Butadien.

Wenn man Butadien als Reaktand und als wesentliche Komponente der hydrophoben Phase verwenden will, kann man es (bezogen auf 1 Mol Ammoniak) beispielsweise in Mengen von mindestens 0,5 Mol einsetzen. Vorzugsweise liegt diese Menge bei 0,7 bis 100 Mol, insbesondere bei 3 bis 50 Mol. Wenn man Butadien nur als Reaktand einsetzt, kann man es (bezogen auf 1 Mol Ammoniak) beispielsweise in Mengen von 0,5 bis 20 Mol, vorzugsweise von 0,7 bis 5 Mol einsetzen.

Als hydrophile Palladiumkomplexe kommen z.B. solche der Formel (I) in Frage.

$$[L^1_x L^2_y Pd]_m^{p+}[A]_n^{q-} \quad (I)$$

, in der

$L^1$ für gleiche oder verschiedene Liganden aus der Gruppe sulfonierter, carboxylierter und hydroxylierter Trialkyl- und Triphenylphosphane und -phosphanoxide,

$L^2$ für gleiche oder verschiedene Liganden aus der Gruppe H, CO, NO, $NH_2$, $NH_3$, $PF_3$, $H_2O$, S, Halogene, aromatische Liganden, olefinische Liganden allylische Liganden und acetylenische Liganden,

x für eine ganze Zahl von Null bis 6 und

y für Null oder eine ganze Zahl von 1 bis 5 stehen,

wobei die Summe aus x + y mindestens 1 und höchstens 6 ergibt und

m für 1, 2 oder 3 und

n, p und q jeweils für Null, 1, 2 oder 3 stehen,

wobei die Beziehung gilt:

$$m \cdot p = n \cdot q \text{ und}$$

A für ein Anion mit der Ladung q steht.

Als Liganden $L^1$ kommen bevorzugt Mono-, Bis- und Tri(hydroxy-$C_1$-$C_5$-alkyl)-phosphane, Mono-, Bis- und Tri(hydroxy-$C_1$-$C_5$-alkyl)-phosphanoxide, mono-, di- und trisulfonierte Triphenylphosphane in der Säureform und mono-, di- und trisulfonierte Triphenylphosphane in der Salzform in Frage. Insbesondere sulfonierte Triphenylphosphan-Liganden können an den Phenylringen noch weitere Substituenten enthalten, beispielsweise pro Triphenylphosphan-Einheit 1 bis 3 gleiche oder verschiedene Halogenatome und/oder 1 bis 3 gleiche oder verschiedene $C_1$-$C_{10}$-Alkylgruppen und/oder 1 bis 3 gleiche oder verschiedene $C_1$-$C_{10}$-Alkoxygruppen.

Besonders bevorzugte Liganden $L^1$ sind Tri(hydroxy-$C_1$-$C_5$-alkyl)-phosphane mit endständiger Anordnung der Hydroxylgruppen an den Alkylresten, Triphenylphosphantrisulfonsäuren, und deren Salze und Triphenylphosphanmono-, -di- und -trimethoxytrinatriumsulfonsäuren, sowie deren Salze und Triphenylphosphanmono-, di- und -trifluormono-, -di- und -trisulfonsäuren, sowie deren Salze.

An Triphenylphosphaneinheiten befindliche Alkylgruppen enthalten vorzugsweise $C_1$-$C_4$-Alkylreste und dort befindliche Alkoxygruppen, vorzugsweise $C_1$-$C_4$-Alkoxyreste.

Ganz besonders bevorzugte Liganden $L^1$ sind Tris(hydroxy-3-propyl)-phosphan sowie das Trinatriumsalz der 3,3',3" Phosphatriyl-tris-(benzolsulfonsäure), im folgenden TPPTS genannt, das Trinatriumsalz der 3,3',3" Phosphatriyl-bis-(benzolsulfonsäure)-mono(4-methoxybenzolsulfonsäure), im folgenden MOM-TPPTS genannt, das Trinatriumsalz der 3,3',3"-Phosphatriylmono(benzolsulfonsäure)-bis-(4-methoxybenzolsulfonsäure), im folgenden BOM-TPPTS genannt, das Trinatriumsalz der 3,3',3"Phosphatriyl-tris-(4-methoxybenzolsulfonsäure), im folgenden TOM-TPPTS genannt, das Trinatriumsalz der 3,3',3"Phosphatriyl-tris-(4-methylbenzolsulfonsäure), im folgenden TOT-TPPTS genannt, das Trinatriumsalz der 5,5',5" Phosphatriyl-tris-(2-fluorbenzolsulfonsäure), im folgenden p-F-TPPTS genannt und das Dinatriumsalz der 5,5'(4-Fluorphenylphosphadiyl)-bis-(2-Fluorbenzolsulfonsäure), im folgenden p-F-TPPDS genannt.

Als Liganden $L^2$ kommen bevorzugt $NH_2$, $NH_3$, Cl, Br, I, Allyl, Methallyl, Cyclopentadienyl, Cyclooctadienyl und Dibenzylidenaceton in Frage.

In Formel (I) steht x vorzugsweise für Null oder eine ganze Zahl von 1 bis 4, insbesondere für eine ganze Zahl von 1 bis 4, und y steht vorzugsweise für Null, 1 oder 2,wobei die Summe von x + y höchstens 4 beträgt.

Als Anion A sind Acetat, Chlorid, Acetylacetonat, Tetrafluoroborat und Hexafluoroantimonat bevorzugt.

Bei MOM-TPPTS und BOM-TPPTS handelt es sich um neue Verbindungen. Diese entsprechen insbesondere den Formeln (II) und (III)

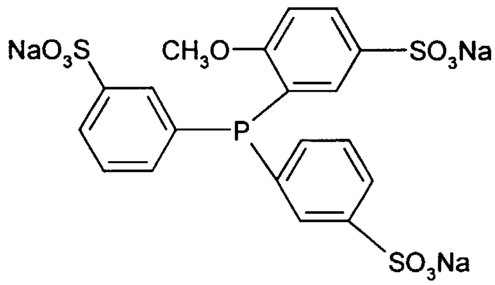

(II)

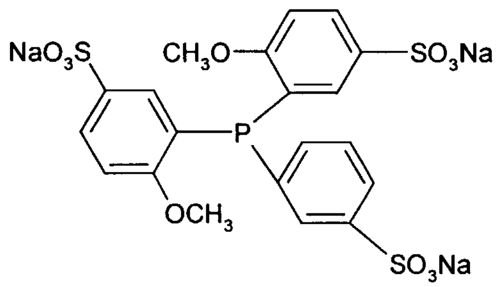

(III)

und sind auch Gegenstand der vorliegenden Erfindung. Ihre Herstellung kann analog Angew. Chemie 107, 893 (1995) erfolgen.

Auch die Verwendung von TOM-TPPTS, TOT-TPPTS, p-F-TPPTS und p-F-TPPDS als Ligand zur Herstellung von Palladiumkomplexen für die Telomerisation von Butadien mit Ammoniak ist neu und Gegenstand der vorliegenden Erfindung. Die Herstellung solcher Verbindungen kann analog Angew. Chemie 107, 893 (1995) oder J. prakt. Chem. 336, 591, (1994) erfolgen.

Die Herstellung der hydrophilen Palladiumkomplexe der Formel (I) kann auf einfache Weise durchgeführt werden, beispielsweise indem man ein Palladiumsalz oder eine Palladiumkomplexverbindung und Wasser vorlegt und Liganden des Typs $L^1$ und/oder des Typs $L^2$ zufügt. Man erhält dann eine wäßrige Lösung des hydrophilen Palladium-Komplexes der Formel (I), die als solche in das erfindungsgemäße Verfahren eingesetzt werden kann, aus der man aber auch die jeweiligen Komplexe der Formel (I) isolieren und dann die isolierten Komplexe in das erfindungsgemäße Verfahren einsetzen kann.

Man kann hydrophile Palladiumkomplexe der Formel (I) auch in-situ herstellen, beispielsweise indem man ein Palladiumsalz oder eine Palladiumkomplexverbindung zusammen mit Wasser oder wäßriger Ammoniaklösung in einem Autoklaven vorlegt, dann Butadien hinzufügt, die gewünschten Liganden und gegebenenfalls weitere wäßrige Ammoniaklösung dazugibt und dann die für die Telomerisation gewünschten Druck- und Temperaturbedingungen einstellt. In diesem Fall kann man beispielsweise pro Mol eingesetztes Palladiumsalz oder Palladiumkomplexbildung 0,1 - 50 Mol des gewünschten Liganden einsetzen. Vorzugsweise beträgt diese Menge 0,2 - 30 Mol, insbesondere 0,3 - 10 Mol. Bei der in-situ Herstellung von Komplexen der Formel (I) können auch Gemische verschiedener Individuen der Formel (I) entstehen, die dann auch in Form solcher Gemische als Katalysatoren für das erfindungsgemäße Verfahren wirksam sind.

Als Ausgangsverbindungen zur Herstellung erfindungsgemäß zu verwendender hydrophiler Palladium-Komplexe können z.B. folgende Palladiumsalze und -komplexe verwendet werden: Palladium(II)-acetat, -chlorid, -acetylacetonat, -hexafluoroantimonat und -tetrafluoroborat sowie Allyldiaminopalladium(II)-tetrafluoroborat, [Bis-(η3-allyl-iodo)-palladium](II), Allyldiaminopalladium(II)-hexafluoroantimonat und beliebige andere Palladium(II)-salze oder -komplexe dieser Art. Die Wahl der Palladiumsalze oder -komplexe zur Herstellung von hydrophilen Palladiumkomplexen der Formel (I) ist nicht von besonderer Bedeutung für das erfindungsgemäße Verfahren. Man kann als Ausgangsverbindungen zur Herstellung erfindungsgemäß zu verwendender hydrophiler Palladiumkomplexe auch Palladium (0)-Komplexe einsetzen, z.B. solche der Formel

$$PdL^1_{x'} \qquad (IV)$$

in der

$L^1$ die bei Formel (I) angegebene Bedeutung hat und

x' für eine ganze Zahl von 1 bis 6 steht,

oder Palladiumdibenzylidenaceton.

Es ist möglich, daß sich die Oxidationstufe des hydrophilen Palladiumkomplexes während der Durchführung des erfindungsgemaßen Verfahrens ändert.

Pro Liter hydrophile Phase kann man beispielsweise 0,5 bis 500 Millimol hydrophile Palladiumkomplexe einsetzen. Vorzugsweise beträgt diese Menge 1 bis 25 Millimol, insbesondere 2,5 bis 10 Millimol. Werden die hydrophilen Palladiumkomplexe in-situ hergestellt, so kann pro Liter hydrophile Phase, beispielsweise 0,5 bis 500 Millimol Palladiumsalz oder Palladiumkomplexverbindung eingesetzt werden. Vorzugsweise beträgt diese Menge 1 bis 25 Millimol, insbesondere 2,5 bis 10 Millimol.

Beim Einsatz von Katalysatoren der Formel (I), bei den $L^1$ für Verbindungen der Formeln (II) oder (III) oder für TOM-TPPTS oder TOT-TPPTS steht, erhält man Reaktionsgemische, die höhere Anteile an Octa-2,7-dienyl-1-amin enthalten. Man kann aber auch hier noch zusätzliches Octa-2,7-dienyl-1-amin erhalten, wenn man das in kleineren Anteilen miterhaltene Octa-1,7-dienyl-3-amin isoliert und zu Octa-2,7-dienyl-1-amin isomerisiert. Der Einsatz von Katalysatoren der Formel (I), bei denen $L^1$ für P-F-TPPTS und p-F-TPPDS steht, führt im allgemeinen zu einer erhöhten Aktivität des Katalysators.

Die Telomerisationsreaktion von Butadien mit Ammoniak kann z.B. bei Temperaturen im Bereich von 30 bis 150°C und Drucken in Bereichen von 1 bis 30 bar durchgeführt werden. Vorzugsweise arbeitet man bei 50 bis 120°C und in einem Autoklaven unter dem sich bei der jeweiligen Reaktionstemperatur von selbst ergebenden Druck.

Die Telomerisationsreaktion ist i.a. nach 30 Minuten bis 20 Stunden beendet. Es ist vorteilhaft während der Reaktion für eine gute Durchmischung des Reaktionsgemisches zu sorgen.

Die Aufarbeitung des Reaktionsgemisches unter getrennter Isolierung des darin enthaltenden Octa-2,7-dienyl-1-amins und des darin enthaltenen Octa-1,7-dienyl-3-amins kann man beispielsweise wie folgt durchführen:

Wenn man mit Butadien als wesentlichem Bestandteil der hydrophoben Phase gearbeitet hat, so entweicht im Überschuß vorhandenes Butadien bei Entspannen auf Normaldruck. Restliches, im Reaktionsgemisch verbleibendes Butadien kann man gegebenenfalls ausblasen, z.B. mit Stickstoff. Man hat dann eine wäßrige, den Katalysator enthaltende Phase und eine organische, die Reaktionsprodukte enthaltende Phase vorliegen, die man nach Trennung wie weiter unter beschrieben aufarbeiten kann. Es ist vorteilhaft, die hydrophile Phase mit einem geeigneten Lösungsmittel zu extrahieren und die extrahierte Phase dann der hydrophoben Phase zur gemeinsamen Aufarbeitung zuzufügen.

Wenn man zur Bildung der hydrophoben Phase ein organisches Lösungsmittel eingesetzt hat, so kann man die hydrophobe Phase von der hydrophilen Phase trennen, die abgetrennte hydrophile Phase gegebenenfalls mit einem mit Wasser nicht mischbaren organischen Lösungsmittel waschen und die Waschflüssigkeit der hydrophoben Phase zufügen.

Die den Katalysator enthaltende hydrophile Phase kann man beliebig verwenden, z.B. im nächsten Ansatz zur erfindungsgemäßen Herstellung von Octa-2,7-dienyl-1-amin (1) oder zur Isomerisierung von isoliertem Octa-1,7-dienyl-3-amin (2).

Die hydrophobe Phase kann man, gegebenenfalls nach dem Abziehen noch vorhandener Lösungsmittel, und gegebenenfalls nach Zusatz eines Radikalfängers (z.B. eines sterisch gehinderten Phenols) einer fraktionierten Destillation unterwerfen. Bei Drucken von beispielsweise 4 bis 40 mbar sind die Siedepunkte von (1) und (2) genügend weit voneinander entfernt, um in Kolonnen mit beispielsweise 50 bis 150 theroretischen Böden und bei Rücklaufverhältnissen von beispielsweise 3:1 bis 15:1 gut voneinander getrennt zu werden. Man kann so eine erste Fraktion erhalten, die (2) und eine zweite Fraktion, die (1) enthält, jeweils in Reinheiten bis zu 99 % und mehr. Es hinterbleiben dann höhersiedende Bestandteile, insbesondere Bis-octa-dienylamine.

Bis zu diesem Verfahrensschritt kan man (1) in Selektivitäten (bezogen auf umgesetztes Butadien) von beispielsweise 40 bis 87 % und (2) in Selektivitäten (bezogen auf umgesetztes Butadien) von beispielsweise 7 bis 40 % erhalten.

Erfindungsgemäß wird anschließend die Selektivität der Bildung von (1) um beispielsweise 1 bis 25 % (bezogen auf die ursprüngliche Selektivität) gesteigert, wenn man das wie oben beschrieben erhaltene (2) einer Isomerisierung unter Bildung von (1) unterzieht.

Diese Isomerisierung kann man analog zu der zuvor beschriebenen Telomerisierung vornehmen, d.h. mit den gleichen Katalysatoren und in einem Zweiphasensystem. Vorteilhafterweise verwendet man für die Isomerisierug den bei der Aufarbeitung des Telomerisationsgemisches abgetrennten Katalysator bzw. dessen Lösung. Abweichend von den oben für die Telomerisation angegebenen Parametern ist es vorteilhaft die Isomerisierung unter folgenden Bedingungen vorzunehmen:

Pro Liter hydrophile Phase kann man beispielsweise 0,5 bis 500 Millimol hydrophile Palladiumkomplexe einsetzen. Vorzugsweise beträgt diese Menge 1 bis 25 Millimol, insbesondere 2,5 bis 10 Millimol. Man kann die hydrophilen Palladiumkomplexe auch in-situ herstellen, beispielsweise indem man ein Palladiumsalz oder eine Palladiumkomplexverbindung zusammen mit Wasser oder einer wäßrigen Ammoniaklösung vorlegt und den gewünschten Ligand zugibt. In diesem Fall kann man pro Liter hydrophile Phase beispielsweise 0,5 bis 500 Millimol Palladiumsalz oder Palladiumkomplex einsetzen. Vorzugsweise beträgt diese Menge 1 bis 25 Millimol, insbesondere 2,5 bis 10 Millimol. Pro Mol eingesetztes Palladiumsalz oder Palladiumkomplexverbindung kann man 0,1 - 50 Mol des gewünschten Liganden einsetzen. Vorzugsweise beträgt diese Menge 0,2 - 30 Mol, insbesondere 0,3 bis 10 Mol.

Als hydrophile Phase kann als wesentliche Komponente z.B. Wasser oder ein hydrophiles Lösungsmittel oder Ammoniak eingesetzt werden. Es können auch beliebige Mischungen aus Wasser, hydrophilen Lösungsmitteln und Ammoniak eingesetzt werden. Dabei ist die Anwesenheit von Ammoniak in jedem Fall bevorzugt. Bei den hydrophilen Lösungsmitteln handelt es sich z.B. um $C_1$-$C_6$ Mono-, Di-, Tri-, Tetra-, Penta- und Hexaalkohole, um Tetramethylensulfolan, Dimethylsulfoxid oder Acetonitril. Als hydrophile Phase werden bevorzugt Wasser, Ammoniak und Mischungen aus Wasser und Ammoniak mit einem Ammoniakgehalt von 1 bis 99 Gew.-% eingesetzt. Besonders bevorzugt ist ein Ammoniakgehalt von 5 bis 60 Gew.-%. Ganz besonders bevorzugt beträgt der Ammoniakgehalt 10 bis 30 Gew.-%. Pro Liter hydrophile Phase können z.B. 10 bis 5 000 g Octa-1,7-dienyl-3-amin (2) eingesetzt werden. Bevorzugt setzt man 50 bis 3 000 g und besonders bevorzugt 100 bis 2 000 g Octa-1,7-dienyl-3-amin ein.

Die Temperatur beträgt z.B. 20 bis 200°C, bevorzugt 40 bis 150°C und besonders bevorzugt 60 bis 120°C.

Der Druck beträgt z.B. 0 bis 500 bar. Bevorzugt wird bei einem Druck von 0 bis 100 bar, besonders bevorzugt bei dem Druck, der sich bei der jeweiligen Temperatur von selbst einstellt, gearbeitet. Es ist auch möglich, die Reaktion in einer Apparatur durchzuführen, in der über einen Druckausgleich mit der Umgebung stets Atmosphärendruck herrscht.

Die Reaktionszeit beträgt im allgemeinen 1 Stunde bis 14 Tage und richtet sich nach dem Verhältnis von Octa-1,7-dienyl-3-amin (2) zu dem eingesetzten Palladiumsalz oder Palladiumkomplex und dem angestrebten Isomerisierungsgrad. Es ist vorteilhaft während der Reaktion für eine gute Durchmischung des Reaktionsgemisches zu sorgen.

Das nach Beendigung der Isomerisierung vorliegende Reaktionsgemisch kann wie das nach der Telomerisierung vorliegende Reaktionsgemisch beispielsweise durch eine Phasenseparation, der Extraktion der wäßrigen Phase durch ein organisches Lösungsmittel und eine anschließende Destillation der vereinigten organischen Phasen aufgearbeitet werden.

Es ist auch möglich, die Isomerisierung mehrere Male hintereinander mit dem aus der jeweils vorgehenden Stufe abgetrennten (2) durchzuführen.

## Beispiele

### Beispiel 1

In einem 500 ml-Dreihalskolben werden unter Argonatmosphäre 4,38 g Borsäure in 41 ml konzentrierter Schwefelsäure vollständig gelöst (durch die Schwefelsäure wird zuvor Argon geleitet, um sie von Sauerstoff zu befreien). Danach werden 5 g *ortho*-Methoxyphenyl)diphenylphosphan darin gelöst und mit einer Eis/Kochsalz-Mischung auf -10°C gekühlt. Bei dieser Temperatur werden über einen Zeitraum von 2 h 73,9 ml Oleum zugetropft und die Lösung 3 d bei Raumtemperatur nachgerührt.

Bevor die Synthese mit der Hydrolyse fortgesetzt wird, nimmt man zunächst eine Probe von 5 ml und arbeitet diese in gleicher Weise auf, wie es im Folgenden für den gesamten Ansatz beschrieben ist. Eine $^{31}$P-NMR-spektroskopische Untersuchung dieser Probe gibt Auskunft darüber, ob die Sulfonierung bereits vollständig verlaufen ist. Bei zu kurzen Reaktionszeiten ist noch mono- und disulfoniertes Produkt, bei zu langen Reaktionszeiten das Phosphanoxid enthalten. Diese Verunreinigungen sind sämtlich sehr schwierig abzutrennen.

Zur Hydrolyse werden unter Eiskühlung sehr langsam 50 ml sauerstofffreies Wasser zugegeben. Anschließend wird der Ansatz in einem 2 l Schlenkkolben überführt und dort unter Eiskühlung mit 7,5 molarer Natronlauge neutralisiert. Es empfiehlt sich den pH-Wert möglichst genau mit Hilfe einer pH-Elektrode bei 7 einzustellen. Die Lösung wird nun am Rotationsverdampfer zur Trockene eingedampft und anschließend das Produkt mit 250 ml Methanol extrahiert. Die Lösung wird über Celite® filtriert und Methanol im Vakuum abdestilliert. Der Rückstand wird mit 20 ml Wasser aufgenommen, über einen Spritzenfilter filtriert und das Wasser im Vakuum enfernt. Durch die abwechselnde Extraktion mit Methanol und Wasser wird MOM-TPPTS als Trihydrat in einer Reinheit von 93 % und einer Ausbeute von 14 % erhalten.

$^{31}$-NMR ($D_2O$): -14,6 ppm
$^{1}$H-NMR ($D_2O$): 3,6 ppm (3H); 6,96 ppm (1H); 7,02 ppm (1H); 7,3 ppm (2H); 7,4 ppm (2H); 7,6 ppm (2H); 7,7 ppm (3H)
$^{13}$C-NMR ($D_2O$): 164,2 ppm; 144,6 ppm; 138,1 ppm; 137,06 ppm; 137,1 ppm; 132,25 ppm; 131,9 ppm; 131,2 ppm; 130,8 ppm; 128,2 ppm; 125,7 ppm; 112,9 ppm; 57,7 ppm

**Beispiel 2**

Die Synthese von BOM-TPPTS wird völlig analog, wie in Beispiel 1 beschrieben durchgeführt, jedoch wird anstelle von (*ortho*-Methoxyphenyl)diphenylphosphan Bis(*ortho*-methoxyphenyl)-phenylphosphan eingesetzt. BOM-TPPTS wird als Trihydrat in 92 %iger Reinheit mit einer Ausbeute von 14 % erhalten.

$^{31}$-NMR ($D_2O$): -24,34 ppm
$^{1}$H-NMR ($D_2O$): 3,6 ppm (6H); 7,0 ppm (2H); 7,03 ppm (2H); 7,3 ppm (1H); 7,4 ppm (1H); 7,6 ppm (1H); 7,7 ppm (3H)
$^{13}$C-NMR ($D_2O$): 161,7 ppm; 141,9 ppm; 135,5 ppm; 134,4 ppm; 133,55 ppm; 129,6 ppm; 129,4 ppm; 128,5 ppm; 128 ppm; 125,6 ppm; 121,8 ppm; 110,3 ppm; 55 ppm

**Beispiel 3**

1 372 mg [Bis-($\eta^3$-allyl-iodopalladium)] werden in 10 ml einer wässrigen 27 %igen $NH_3$-Lösung gelöst. Diese Lösung gibt man zu einer Lösung von 973,4 mg $AgBF_4$ in 5 ml Wasser. Das dabei ausfallende AgI wird anschließend über Celite® abfiltriert. Das Filtrat wird eingedampft und aus dem Rückstand das Produkt durch dreimalige Extraktion mit jeweils 10 ml siedendem $CH_2Cl_2$ herausgelöst und diese Lösung jeweils abdekantiert. Beim Auskühlen fällt bereits ein Teil des Produktes aus, der übrige Teil wird durch die Zugabe von 20 ml Pentan ausgefällt. Der weiße Feststoff wird drei mal mit jeweils 5 ml Pentan gewaschen und im Hochvakuum getrocknet. [$^3\eta$-Allyl-diaminopalladium]-tetrafluoroborat wird in einer Ausbeute von 50,3 % erhalten.

$^{1}$H-NMR ($D_2O$): 2,9 ppm (2H); 4,0 ppm (2H); 5,5 ppm (1H)
$^{13}$C-NMR ($D_2O$): 61 ppm; 118 ppm

**Beispiel 4**

Die Reaktion wird in einem Edelstahlautoklaven mit einem Volumen von 125 ml durchgeführt, der ein Rührsystem aus einem 6-Blatt-Scheibenrührer in Kombination mit Strombrechern enthält und elektrisch beheizt ist. Der Autoklav wird vormontiert und zur Entfernung des Sauerstoffs drei mal evakuiert und mit Argon befüllt.

In ein Schlenkrohr werden 30,3 mg [$^3\eta$-Allyl-diaminopalladium]-tetrafluoroborat eingewogen und mit 15 ml einer wäßrigen 27 %igen Ammoniaklösung in den Autoklaven überfuhrt. Anschließend werden über eine Dosierkartusche 40 g Butadien eingefüllt. Der Reaktorinhalt wird unter Rühren (2000 rpm) auf 80°C aufgeheizt. Währenddessen werden 0,113 mMol TPPTS in Form einer 32,4 %igen wäßrigen Lösung in ein Schlenkrohr eingewogen und mit 15 ml einer wäßrigen Ammoniaklösung in einen Stahltropftrichter überführt. Dieser wird an den Autoklaven montiert. Bei einer Innentemperatur von 80°C wird zunächst der Druckausgleich und dann das Auslaufventil des Tropftrichters geöffnet. Die Temperatur fällt auf ca. 60°C ab, erreicht aber binnen 3 min wieder den Sollwert von 80°C. Nach 45 min Reaktionszeit wird der Autoklav in ein Eisbad gesetzt und bei verminderter Drehzahl des Rührers (200 rpm) das überschüssige Butadien über einen Bunsenbrenner abgelassen. Auf diese Weise kühlt sich der Reaktorinhalt innerhalb von 5 min auf 20°C ab. Wenn das Butadien vollständig abgefackelt ist, wird der Autoklav geöffnet und der Inhalt in einen Scheidetrichter überführt. Es wird mit 10 ml Toluol nachgespült und extrahiert und eine Spatelspitze Kochsalz zur Beschleunigung der Phasentrennung zugegeben. Nach der Phasentrennung wird in die organische Phase der GC-Standard Undecan eingewogen und diese über Molsieb 4 Å getrocknet. Anschließend wird das Molsieb abfiltriert und mit 10 ml Toluol nachgewaschen. Es wird eine Probe entnommen und die Zusammensetzung gaschromtographisch untersucht. Der Umsatz bezüglich Butadien betrug 5,8 %. Die Zusammensetzung der Probe war 76 % (1), 21 % (2) und 3 % (3).

**Beispiele 5 bis 7**

Die Vorgehensweise ist identisch mit der in Beispiel 4 beschriebenen. Hier werden allerdings anstelle 0,113 mMol TPPTS andere Stoffmengen dieser Verbindung eingesetzt. Die Beispiele sind in Tabelle 1 zusammengestellt:

Tabelle 1

| Beispiel Nr. | Stoffmenge TPPTS | Umsatz (bezogen auf Butadien) | Zusammensetzung (Gew.-%) | | |
|---|---|---|---|---|---|
| | | | (1) | (2) | (3) |
| 5 | 0,226 mmol | 15,5 % | 50 | 35 | 12 |
| 6 | 0,452 mmol | 7,0 % | 51 | 43 | 4 |
| 7 | 0,678 mmol | 0,9 % | 48 | 50 | 0,5 |

**Beispiele 8 bis 11**

Die Vorgehensweise ist identisch mit der in Beispiel 4 beschriebenen. Hier wird allerdings anstelle einer wäßrigen Lösung von TPPTS MOM-TPPTS in Form des Trihydrates eingesetzt. Die eingesetzten Stoffmengen dieser Verbindung werden ebenfalls variiert. Die Beispiele sind in Tabelle 2 zusammengestellt:

TABELLE 2

| Beispiel Nr. | Stoffmenge MOM-TPPTS | Umsatz (bezogen auf Butadien) | Zusammensetzung (Gew.-%) | | |
|---|---|---|---|---|---|
| | | | (1) | (2) | (3) |
| 8 | 0,113 mmol | 6 % | 70 | 22 | 7 |
| 9 | 0,226 mmol | 16,5 % | 49 | 40 | 8 |
| 10 | 0,452 mmol | 13,4 % | 40 | 37 | 8 |
| 11 | 0,678 mmol | 5,2 % | 42 | 40 | 2,2 |

**Beispiele 12 bis 15**

Die Vorgehensweise ist identisch mit der in Beispiel 4 beschriebenen. Hier wird anstelle einer wäßrigen Lösung von TPPTS BOM-TPPTS in Form seines Trihydrates eingesetzt. Die eingesetzten Stoffmengen dieser Verbindung werden ebenfalls variiert. Die Beispiele sind in Tabelle 3 zusammengestellt:

Tabelle 3

| Beispiel Nr. | Stoffmenge BOM-TPPTS | Umsatz (bezogen auf Butadien) | Zusammensetzung (Gew.-%) | | |
|---|---|---|---|---|---|
| | | | (1) | (2) | (3) |
| 12 | 0,113 mmol | 2,9 % | 87 | 7 | 5 |
| 13 | 0,226 mmol | 6,5 % | 65 | 17 | 0,3 |
| 14 | 0,452 mmol | 8,0 % | 58 | 33 | 7 |
| 15 | 0,678 mmol | 9,3 % | 53 | 33 | 7 |

**Beispiele 16 bis 19**

Die Vorgehensweise ist identisch mit der in Beispiel 4 beschriebenen. Hier wird anstelle einer wäßrigen Lösung von TPPTS TOM-TPPTS in Form seines Trihydrates eingesetzt. Die eingesetzten Stoffmengen dieser Verbindung werden ebenfalls variiert. Die Beispiele sind in Tabelle 4 zusammengestellt:

Tabelle 4

| Beispiel Nr. | Stoffmenge TOM-TPPTS | Umsatz (bezogen auf Butadien) | Zusammensetzung (Gew.-%) | | |
|---|---|---|---|---|---|
| | | | (1) | (2) | (3) |
| 16 | 0,113 mmol | 1,05 % | 94 | 3 | 1 |
| 17 | 0,226 mmol | 2,25 % | 94 | 3,5 | 1,2 |
| 18 | 0,452 mmol | 2,3 % | 87 | 4 | 3 |
| 19 | 0,678 mmol | 2,3 % | 89 | 7 | 3 |

**Beispiele 20 bis 22**

Die Vorgehensweise ist identisch mit der in Beispiel 4 beschriebenen. Hier wird allerdings anstelle einer wäßrigen Lösung von TPPTS TOT-TPPTS in Form seines Trihydrates eingesetzt. Die eingesetzten Stoffmengen dieser Verbindung werden ebenfalls variiert. Die Beispiele sind in Tabelle 5 zusammengestellt:

Tabelle 5

| Beispiel Nr. | Stoffmenge TOT-TPPTS | Umsatz (bezogen auf Butadien) | Zusammensetzung (Gew.-%) | | |
|---|---|---|---|---|---|
| | | | (1) | (2) | (3) |
| 20 | 0,113 mmol | 1,05 % | 91 | 5 | 1 |
| 21 | 0,339 mmol | 3,3 % | 86 | 4 | 8 |
| 22 | 0,678 mmol | 2,7 % | 90 | 5 | 3 |

**Beispiel 23**

Die Vorgehensweise ist identisch mit der in Beispiel 4 beschriebenen. Hier werden allerdings anstelle einer wäßrigen Lösung von TPPTS 2,428 mmol TOM-TPPTS in Form des Trihydrates und 0,971 mol [$^{3}\eta$-Allyl-diaminopalladium]-tetrafluoroborat eingesetzt. Der Umsatz bezüglich Butadien betrug 29,0 %. Die Zusammensetzung der Probe ist 67 Gew.- (1); 13 Gew.-% (2) und 17 Gew.-% (3).

**Beispiel 24**

Die Vorgehensweise ist identisch mit der in Beispiel 18 beschriebenen. Hier wird allerdings eine Reaktionszeit von 7 Stunden eingehalten. Der Umsatz bezüglich Butadien betrug 30,4 %. Die Zusammensetzung der Probe ist 78 Gew.- (1); 5 Gew.-% (2) und 16 Gew.-% (3).

**Beispiel 25**

Die Vorgehensweise ist identisch mit der in Beispiel 4 beschriebenen. Hier wird allerdings anstelle einer wäßrigen Lösung von TPPTS eine Mischung aus 0,099 mmol p-F-TPPDS in Form des Dihydrates und 0,015 mmol p-F-TPPTS in Form des Trihydrates eingesetzt. Der Umsatz bezüglich Butadien betrug 8,2 %. Die Zusammensetzung der Probe ist 54 Gew.- (1); 38 Gew.-% (2) und 6 Gew.-% (3).

**Beispiele 26 bis 29**

Die Vorgehensweise ist identisch mit der in Beispiel 4 beschriebenen. Hier werden allerdings anstelle [$^{3}\eta$-Allyl-diaminopalladium]-tetrafluoroborat andere Palladiumverbindungen eingesetzt. Außerdem wurde die eingesetzte Stoffmenge des TPPTS variiert. Die Beispiele sind in Tabelle 6 zusammengestellt:

Tabelle 6

| Beispiel Nr. | Palladiumverbindung | Stoffmenge TPPTS | Umsatz (bezogen auf Butadien) | Zusammensetzung (Gew.-%) | | |
|---|---|---|---|---|---|---|
| | | | | (1) | (2) | (3) |
| 26 | Palladiumacetat | 0,339 mmol | 15,7 % | 53 | 35 | 6 |
| 27 | Palladiumbis-(diacetylacetonat) | 0,339 mmol | 11,3 % | 53 | 40 | 6 |
| 28 | Palladiumchlorid | 0,339 mmol | 14,2 % | 54 | 39 | 6 |
| 29 | [Bis-($\eta^3$-allyl-iodo-palladium)] | 0,226 mmol | 13,5 % | 58 | 32 | 9 |

**Beispiel 30**

In einen zuvor drei mal evakuierten und mit Argon befüllten Edelstahlautoklaven wird im Argongegenstrom eine Lösung von 1,5 mmol Palladiumacetat und 4,5 mml TPPTS (TPPTS wird in Form einer 32,4 %igen wäßrigen Lösung eingesetzt) in 250 ml einer 20 %igen wäßrigen Ammoniaklösung vorgelegt. Hierzu werden 120 g Butadien einkondensiert und der Autoklav unter heftigem Rühren über einen Zeitraum von 15 min auf 80°C aufgeheizt. Die Reaktionsmischung wird bei dieser Temperatur weitere 45 min intensiv durchmischt. Danach wird die Heizung abgestellt und das überschüssige Butadien über einen Bunsenbrenner verbrannt. Dabei kühlt sich der Inhalt des Autoklaven ab. Wenn das gesamte Butadien verbrannt ist wird der Inhalt des Autoklaven in einen Scheidetrichter überführt und die Phasen getrennt. Die wäßrige Phase wird 3 mal mit je 40 ml Pentan extrahiert und dieser Extrakt mit der organischen Phase vereinigt. Das sich dabei abscheidende Wasser wird abgetrennt und verworfen. Die verbleibende organische Phase wird über Molsieb getrocknet. Das Trockenmittel wird abgetrennt und das Lösungsmittel am Rotationsverdampfer entfernt. Die verbleibende Flüssigkeit wird mit 2 g 2,4-Di-*tert*.-Butylphenol versetzt und einer fraktionierten Destillation unterworfen. Dabei wird eine Kolonne mit 80 theoretischen Böden bei einem Rücklaufverhältnis von 1:10 eingesetzt. Bei einem Druck von 20 mbar werden bei einer Kopftemperatur von 61°C 34,1 g der Verbindung (2) isoliert. Ebenfalls bei 20 mbar werden bei einer Kopftemperatur von 75,5°C 55 g der Verbindung (1) isoliert.

Die gesamte Menge der Verbindung (2) wird zur Isomerisierung zusammen mit 0,974 mmol Palladiumacetat, 5,58 mmol TPPTS (in Form einer 32,4 %igen wäßrigen Lösung) und 290 ml einer 27 %igen Ammoniaklösung in einen Glasautoklaven überführt. Die Mischung wird 15 Stunden lang bei einer Temperatur von 80°C heftig gerührt und anschließend auf Raumtemperatur gekühlt. Die Phasen werden getrennt, die wäßrige Phase mit insgesamt 50 ml Pentan extrahiert und die Extrakte mit der organischen Phase vereinigt. Das dabei abgeschiedene Wasser wird abgetrennt und verworfen. Die organische Phase wird über Molsieb getrocknet, das Trockenmittel abgetrennt und das Lösungsmittel am Rotationsverdampfer entfernt. Der Rückstand wird, wie bereits oben beschrieben einer fraktionierten Destillation unterworfen. Man erhält 19,8 g der Verbindung (2) und 6,5 g der Verbindung (1). Die gesamte Menge der Verbindung (2) wird völlig analog, wie zuvor beschrieben erneut einer Isomerisierung und einer anschließenden destillativen Trennung unterworfen. Hierbei erhält man 3,6 g der Verbindung (1) und 11,5 g der Verbindung (2). Wiederum wird die gesamte Menge der Verbindung (2) völlig analog, wie zuvor beschrieben einer Isomerisierung und einer anschließenden destillativen Trennung unterworfen. Hierbei erhält man 1,8 g der Verbindung (1) und 6,1 g der Verbindung (2).

Insgesamt wurden durch die hier beschriebene Telomerisation mit anschließender dreimaliger Isomerisierung 66,9 g der Verbindung (1) erhalten, was einen Umsatz von 48,2 % bezüglich Butadien entspricht.

**Beispiel 31 und 32**

Die Versuche zur Rezirkulierung der Katalysatorphase wurden in einem Edelstahlautoklaven mit einem Volumen von 125 ml durchgeführt, der ein Rührsystem aus einem 6-Blatt-Scheibenrührer in Kombination mit Strombrechern enthält und elektrisch beheizt ist. Der Autoklav wird vormontiert und zur Entfernung des Sauerstoffs drei mal evakuiert und mit Argon befüllt.

In ein Schlenkrohr werden 25,4 mg Palladiumacetat und die entsprechende Menge TPPTS eingewogen. Der Katalysator wird mit 30 ml einer 27 %igen wäßrigen $NH_3$-Lösung in den Autoklaven überführt. Anschließend werden 40 g Butadien einkondensiert und der Autoklav unter Rühren auf Reaktionstemperatur (80°C) aufgeheizt. Die Aufheizphase dauert 25 min nach dieser Aufheizphase wird die Reaktionstemperatur weitere 50 min gehalten. Danach wird der Autoklav in ein Eisbad gesetzt und das Butadien über einen Bunsenbrenner abgelassen. Dabei geht die Innentemperatur binnen 5 min auf 20°C zurück. Wenn das Butadien vollständig abgefackelt ist, wird der Reaktorinhalt über ein Steigrohr, das im Inneren des Autoklaven bis auf den Grund geht, in einen Schutzgasscheidetrichter überführt. Dazu wird über das Nadelventil Argon mit einem Druck von 0,5 bar auf den Autoklaven gegeben und so die Flüssigkeit über das Steigrohr hinausgepreßt. Im Scheidetrichter werden die Phasen getrennt und die Katalysatorphase wieder in den Autoklaven überführt. In den Autoklaven werden nun 1 g Ammoniak und 40 g Butadien einkondensiert. Ab jetzt wird verfahren, wie für den ersten Lauf bereits beschrieben. Insgesamt werden 4 Läufe mit der gleichen Katalysatorphase durchgeführt. Die organischen Phasen werden mit 10 ml Toluol versetzt und der GC-Standard Undecan eingewogen. Anschließend werden sie über Molsieb 4 Å getrocknet und die Zusammensetzung gaschromatographisch bestimmt. Die Ergebnisse sind in den Tabellen 7 und 8 zusammengefaßt.

Tabelle 7

| Beispiel 31: 0,339 mmol TPPTS wurden eingesetzt | | | | |
|---|---|---|---|---|
| | Umsatz (bezogen auf Butadien) | Zusammensetzung (Gew.-%) | | |
| | | (1) | (2) | (3) |
| Lauf 1 | 17,6 % | 55 | 37 | 7 |
| Lauf 2 | 14,3 % | 53 | 36 | 10 |
| Lauf 3 | 12,9 % | 53 | 31 | 15 |
| Lauf 4 | 5,9 % | 54 | 28 | 16 |

Tabelle 8

| Beispiel 32: 0,565 mmol TPPTS wurden eingesetzt | | | | |
|---|---|---|---|---|
| | Umsatz (bezogen auf Butadien) | Zusammensetzung (Gew.-%) | | |
| | | (1) | (2) | (3) |
| Lauf 1 | 4,3 % | 50 | 45 | 3 |
| Lauf 2 | 6,6 % | 49 | 43 | 5 |
| Lauf 3 | 9,2 % | 47 | 40 | 8 |
| Lauf 4 | 8,1 % | 46 | 41 | 7 |

**Beispiel 33**

Es wurde verfahren, wie in den Beispielen 31 und 32 beschrieben, allerdings wurde anstelle von TPPTS TOM-TPPTS eingesetzt. Eine eingewogene Menge TOM-TPPTS entsprach einer Stoffmenge von 0,565 mmol. Die Reaktionszeit betrug 200 Minuten. Das Ergebnis des Versuchs ist in Tabelle 9 zusammengestellt.

Tabelle 9

| | Umsatz (bezogen auf Butadien) | Zusammensetzung (Gew.-%) | | |
|---|---|---|---|---|
| | | (1) | (2) | (3) |
| Lauf 1 | 7,7 % | 88 | 45 | 6,5 |
| Lauf 2 | 10,7 % | 85 | 5 | 10 |

Tabelle 9 (fortgesetzt)

| | Umsatz (bezogen auf Butadien) | Zusammensetzung (Gew.-%) | | |
|---|---|---|---|---|
| | | (1) | (2) | (3) |
| Lauf 3 | 9,6 % | 77 | 5 | 16 |
| Lauf 4 | 8,3 % | 75 | 5 | 17 |

## Patentansprüche

1. Verfahren zur Herstellung von Octa-2,7-dienyl-1-amin durch Telomerisation von Butadien mit Ammoniak in einem Zweiphasensystem in Gegenwart von hydrophilen Palladiumkomplexen, dadurch gekennzeichnet, daß man aus dem nach der Telomerisation vorliegenden Reaktionsgemisch getrennt voneinander Octa-2,7-dienyl-1-amin und Octa-1,7-dienyl-3-amin isoliert und das so isolierte Octa-1,7-dienyl-3-amin einer Isomerisierung unter Bildung von Octa-2,7-dienyl-1-amin unterwirft.

2. Verfahren nach Anspruch 1, dadurch gekennzeichnet, daß man die Katalysatorphase nach der Reaktion abtrennt und erneut einsetzt.

3. Verfahren nach Ansprüchen 1 und 2, dadurch gekennzeichnet, daß man als hydrophile Palladiumkomplexe solche der Formel (I)

$$[L^1_x L^2_y Pd]_m^{p+} [A]_n^{q-} \qquad (I)$$

, in der

$L^1$ für gleiche oder verschiedene Liganden aus der Gruppe sulfonierter, carboxylierter und hydroxylierter Trialkyl- und Triphenylphosphane und -phosphanoxide,

$L^2$ für gleiche oder verschiedene Liganden aus der Gruppe H, CO, NO, $NH_2$, $NH_3$, $PF_3$, $H_2O$, S, Halogene, aromatische Liganden, olefinische Liganden allylische Liganden und acetylenische Liganden,

x für eine ganze Zahl von Null bis 6 und

y für Null oder eine ganze Zahl von 1 bis 5 stehen,

wobei die Summe aus x + y mindestens 1 und höchstens 6 ergibt und

m für 1, 2 oder 3 und

n, p und q jeweils für Null, 1, 2 oder 3 stehen,

wobei die Beziehung gilt:

$$m \cdot p = n \cdot q \text{ und}$$

A für ein Anion mit der Ladung q steht.

4. Verfahren nach Ansprüchen 1 bis 3, dadurch gekennzeichnet, daß man zur Herstellung des Katalysators als Palladiumsalze oder Palladiumkomplexe Palladium(II)-acetat, Palladium(II)-chlorid, Palladium(II)acetylacetonat oder Bis-(allyliodo)-palladium(II) einsetzt.

5. Natriumsalz der 3,3',3"Phosphantriyl-bis-(benzolsulfonsäure)-mono(4-methoxybenzolsulfonsäure) und das Natriumsalz der 3,3',3"Phosphantriylmono-(benzolsulfonsäure)-bis(4-methoxy-benzolsulfonsäure der Formeln (II) und (III)

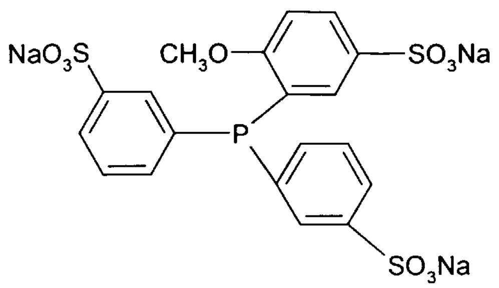

(II)

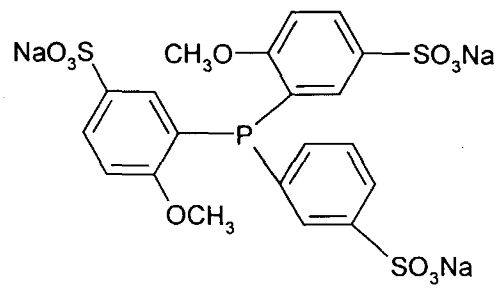

(III).

6. Verwendung von dem Trinatriumsalz der 3,3',3"Phosphantriyl-tris-(4-methoxybenzolsulfonsäure), dem Trinatriumsalz der 3,3',3"-Phosphatriyl-tris-(4-methylbenzol-sulfonsäure, dem Trinatriumsalz der 5,5',5"-Phosphatriyl-tris-(2-fluorbenzolsulfonsäure) und dem Dinatriumsalz der 5,5'(4-Fluorphenylphosphadiyl)-bis-(2-fluorbenzolsulfonsäure) als Liganden zur Herstellung von Palladiumkomplexen für die Telomerisation von Butadien und Ammoniak.

7. Allyldiaminopalladium(II)-tetrafluoroborat.

8. Verwendung von Allyldiamonopalladium(II)-tetrafluoroborat als Vorläufer zur Herstellung von Palladiumkomplexen mit dreiwertigen Phosphor-Verbindungen für die Telomerisation von Butadien und Ammoniak.